(19) Europäisches Patentamt
European Patent Office
Office européen des brevets

(11) Publication number : **0 537 947 A1**

(12) **EUROPEAN PATENT APPLICATION**

(21) Application number : **92309161.5**

(51) Int. Cl.[5] : **C07F 9/12,** A61K 31/66

(22) Date of filing : **06.10.92**

(30) Priority : **09.10.91 GB 9121316**

(43) Date of publication of application : **21.04.93 Bulletin 93/16**

(84) Designated Contracting States : **AT BE CH DE DK ES FR GB GR IE IT LI LU MC NL PT SE**

(71) Applicant : **THE WELLCOME FOUNDATION LIMITED
Unicorn House 160 Euston Road
London NW1 2BP (GB)**

(72) Inventor : **Hudson, Alan Thomas
Langley Court
Beckenham, Kent BR3 3BS (GB)**

(74) Representative : **Rollins, Anthony John et al
Group Patents & Agreements The Wellcome Foundation Ltd Langley Court
Beckenham Kent BR3 3BS (GB)**

(54) **Naphthoquinone derivatives for the treatment of parasitic infections.**

(57) The present invention relates to novel naphthoquinones of formula (I) :

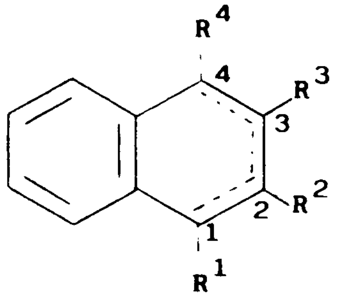

(I)

wherein $R^3$ is a $C_{1-35}$ non-aromatic hydrocarbon residue optionally substituted by one or more substituents selected from halo, $C_{1-6}$ alkoxy, hydroxy, phenyl, phenyl-$C_{1-6}$alkoxy and phenyl-$C_{1-6}$alkyl, each such phenyl group or mainly being optionally substituted by one or more groups selected from $C_{1-6}$ alkoxy, $C_{1-6}$ alkyl, $C_{1-6}$alkoxy-$C_{1-6}$alkyl, hydroxy, halogen, halo-$C_{1-6}$alkyl, amine and mono- or di-$C_{1-4}$alkyl-amino ; and

either the dotted line represents a double bond between the 2 and 3 positions of the naphthyl ring, $R^1$ and $R^4$ each represent =O, and $R^2$ represents a phosphate group

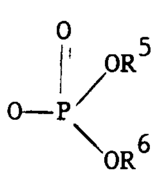

wherein $R^5$ and $R^6$ which may be the same or different, each represent a hydrogen atom or a $C_{1-6}$ alkyl group ;

or the dotted line represents double bonds between the 1,2 and 3,4 positions of the naphthyl ring, and $R^1$, $R^2$ and $R^4$ each represent a phosphate group

$$-OP(O)(OR^5)(OR^6)$$

(wherein $R^5$ and $R^6$ are as hereinbefore described) and physiologically acceptable salts thereof.

The compounds of formula (I) are useful in the treatment of parasitic infections.

EP 0 537 947 A1

The present invention relates to naphthoquinones and their use in chemotherapy. More specifically the invention is concerned with novel phosphate derivatives of hydroxynaphthoqulnones, processes for their preparation, pharmaceutical formulations thereof and their use in the chemotherapy of certain protozoal and parasitic infections.

Parasitic protozoal infections are responsible for a wide variety of diseases of medical and veterinary importance, including malaria in man and various coccidioses in birds, fish and mammals. Many of the diseases are life-threatening to the host and cause considerable economic loss in animal husbandry. Parasitic protozoa include the Apicomplexa, such as species of Eimeria, Cryptosporldium, Toxoplasma, and Plasmodium. Another parasitic organism of increasing concern is Pneumocystis carinii, which can cause an often-fatal pneumonia in immunodeficient or immunocompromised hosts, including those infected with HIV. The classification of this organism is unclear and there is still uncertainty as to whether it is a protozoan or a fungus.

A wide range of naphthoquinones is known in the art. Such compounds have been variously described as having antimalarial, anticoccidial and antitheilerial activity. Some compounds have also been described as possessing activity against external parasites. Thus, Fieser et al, J. Amer. Chem. Soc. 1948, 70, 3156-3165 (and references cited therein) describes a large number of 2-substituted-3-hydroxy-1,4-naphthoquinones as having antimalarial activity. A number of these compounds have also been described in U.S. Patent Specification No. 2 553 648. Further classes of 2-substituted-3-hydroxy-1,4-naphthoquinones having activity as antimalarial, anticoccidial and/or antitheilerial agents are described in U.S. Patents Nos. 3 367 830, and 3 347 742, U.K. Patent Specification No. 1553424, and European Patent Specifications Nos. 2 228, 77551, 77550 and 123,238.

European Patent Application No. 362996 discloses, for the treatment and/or prophylaxis of infections caused by Pneumocystis carinii, 1,4-naphthoquinones of formula:

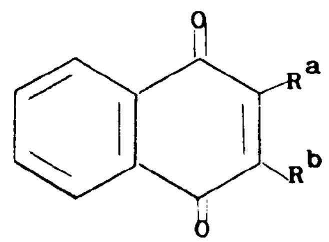

where $R^a$ is an optionally substituted, $C_{1-35}$ non-aromatic hydrocarbon residue and $R^b$ is inter alia a group -$OCOR^c$, $OR^d$, $SR^d$ or $NR^eR^f$, which compounds are said to be believed to act as pro-drugs of compounds wherein $R^b$ is a hydroxyl group.

It has now been found that a 1,4-naphthoquinone substituted by a phosphate group exhibits good activity in vivo against malaria in mice infected with Plasmodium yoelii.

Thus, in a first aspect the present invention provides a compound of general formula (I)

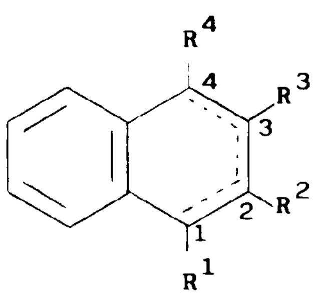

(I)

wherein

$R^3$ is $C_{1-35}$ non-aromatic hydrocarbon residue optionally substituted by one or more substituents, selected from halo, $C_{1-6}$ alkoxy, hydroxy, phenyl, phenyl-$C_{1-6}$ alkoxy and phenyl-$C_{1-6}$ alkyl, each such phenyl group or moiety being optionally substituted by one or more groups selected from $C_{1-6}$alkoxy, $C_{1-6}$alkyl, $C_{1-6}$alkoxy-$C_{1-6}$alkyl, hydroxy, halogen, halo-$C_{1-6}$alkyl, amino, and mono-or di-$C_{1-4}$alkyl-amino; and

either

the dotted line represents a double bond between the 2 and 3 positions of the naphthyl ring,

$R^1$ and $R^4$ each represent = O, and
$R^2$ represents a phosphate group

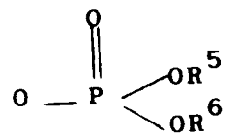

wherein $R^5$ and $R^6$, which may be the same or different, each represent a hydrogen atom or a $C_{1-6}$ alkyl group;
or the dotted line represents double bonds between the 1, 2 and 3, 4 positions of the naphthyl ring, and
$R^1$, $R^2$ and $R^4$ each represent a phosphate group

$-OP(O)(OR^5)(OR^6)$

(wherein $R^5$ and $R^6$ are as hereinbefore defined);

and physiologically acceptable salts thereof. A $C_{1-35}$ non-aromatic hydrocarbon residue $R^3$ may be a straight or branched chain $C_{1-14}$ (e.g. $C_{1-8}$)alkyl or $C_{2-14}$ (e.g. $C_{2-8}$)alkenyl group or a $C_{3-10}$(e.g. $C_{3-8}$)cycloalkyl group, each of which may optionally carry a $C_{3-10}$(e.g. $C_{3-6}$)cycloalkyl group, and each of the aforesaid cycloalkyl groups optionally carrying a $C_{1-10}$ e.g. ($C_{1-4}$)alkyl group. The non-aromatic hydrocarbon residue $R^3$ preferably contains from 1 to 20 carbon atoms, e.g. 1 to 14 carbon atoms. Suitable residues $R^3$ include $C_{3-10}$cycloalkyl-$C_{1-8}$-alkyl, $C_{1-18}$alkyl-$C_{3-10}$cycloalkyl, $C_{1-10}$alkyl-$C_{3-10}$cyclo-alkyl-$C_{1-10}$alkyl and $C_{3-10}$-cycloalkyl-$C_{3-10}$cycloalkyl. $R^3$ may also be substituted as hereibefore defined.

Preferred groups $R^3$ include:

a $C_{1-10}$alkyl group;

a $C_{5-7}$ cycloalkyl group (which may be optionally substituted by a straight or branched chain $C_{1-6}$ alkyl group, a halo-$C_{1-6}$alkyl group, a $C_{1-6}$alkoxy group or a phenyl group, the phenyl group itself being optionally substituted by one or more substituents selected from $C_{1-6}$ alkyl and halogen); and

a $C_{1-10}$alkyl-$C_{5-7}$cycloalkyl group, wherein the cycloalkyl moiety may be optionally substituted as defined for the aforementioned $C_{5-7}$ cycloalkyl group.

A particularly preferred class of substituents $R^3$ is represented by the formula:

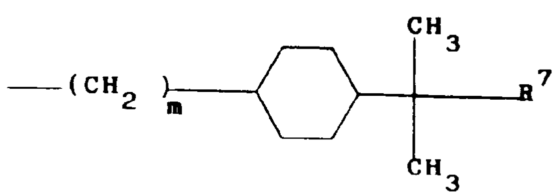

wherein:
m is zero or one and
$R^7$ is a $C_{1-10}$ alkyl group.

Another particularly preferred class of substituents $R^3$ is represented by the formula:

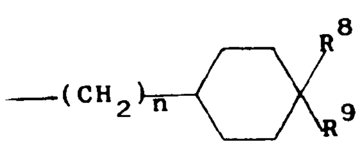

wherein:
n is zero or one, and
either
$R^8$ is hydrogen and
$R^9$ is selected from halogen, perhalo-$C_{1-6}$ alkyl, $C_{1-6}$ alkoxy, aralkoxy, $C_{1-6}$ alkyl-$C_{1-6}$ alkoxy, and phenyl substituted by one or two groups selected from halogen and $C_{1-6}$ alkyl
or

$R^8$ and $R^9$ are both $C_{1-6}$ alkyl or phenyl.

It will be appreciated that the compounds of formula (I) wherein $R^3$ contains a substituted cyclohexyl group may exist as the cis or trans isomer, that is to say that the cyclohexyl ring may be cis or trans substituted by the naphthoquinone nucleus and the substituent on the cyclohexyl ring. Both cis and trans isomers and mixtures thereof in any ratio may be used in accordance with the present invention. In general when the compound is in the form of a mixture of isomers the trans isomer will be present in an amount of about 50% or will be the predominant isomer but the use of mixtures in which the cis isomer predominates is also included within the scope of the invention. The specific ratio of isomers may be varied as required; typical mixtures include those in which the cis/trans isomer ratio is about 1:1,40:60 and 5:95. For use according to the present invention the trans isomer of such compounds of formula (I), or a mixture of the cis and trans isomers containing at least 95% e.g. 99% of the trans isomer, is preferred.

An especially preferred substituent $R^3$ is the 4-(4-chlorophenyl)cyclohexyl group:

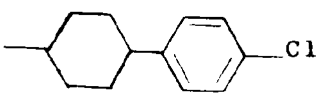

in particular in the trans form with respect to the naphthyl ring.

When either of the groups $R^5$ or $R^6$ represents $C_{1-6}$ alkyl, this may be straight or branched chain e.g. methyl, ethyl, isopropyl, t-butyl, isopentyl or n-hexyl. Preferably $R^5$ and $R^6$ both represent hydrogen atoms.

In the compounds of formula (I) $R^1$ and $R^4$ preferably represent =O and the dotted line is a bond between the 2 and 3 positions of the naphthyl ring.

When either $R^1$ and/or $R^2$ represents hydrogen, a phosphate of formula (I) may form salts with bases. The salts are preferably physiologically acceptable and thus include organic base salts for example formed with primary, secondary or tertiary amines such as ethanolamine, diethanolamine, phenylethylbenzylamine, dibenzylethylenediamine, ammonia, methylamine, dimethylamine, N-methylglucosamine (meglumine), triethylamine, piperidine, and cyclohexylamine; amino acid salts e.g. lysine and arginine; and inorganic base salts such as alkali metal (e.g. sodium and potassium) and alkaline earth metal (e.g. calcium) salts.

A preferred group of compounds of formula (I) may be represented by the formula (II)

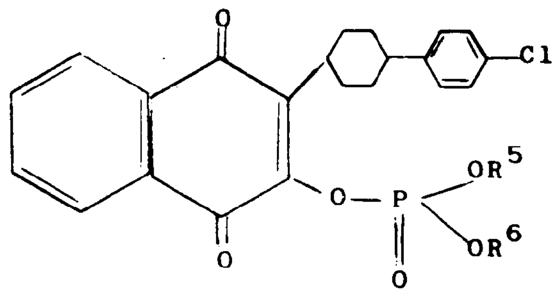

(II)

wherein $R^5$ and $R^6$ are as hereinbefore defined,

and physiologically acceptable salts thereof.

Furthermore the compounds of formula (II) are preferably in the form of the trans-isomer.

A preferred compound according to the present invention is the meglumine salt of the compound (II) wherein both $R^5$ and $R^6$ represent hydrogen. This compound is readily soluble in cold water, and thus has the potential for intravenous administration, as an aqueous solution. A further water soluble salt of interest is the diethanolamine salt.

Without wishing to be bound by theory, it is believed that the phosphate derivatives of formula (I) are prodrugs of the corresponding hydroxynaphthoquinone, that is, the phosphate group is cleaved in vivo to give the compound of formula (I).

It is believed that the compounds of formula (I) will exhibit activity against parasitic protozoa, in particular those organisms against which corresponding hydroxynaphthoquinones have been found to be active, such as Plasmodium species, eg. P.falciparum; Eimeria species eg. E.tenella and E.acervulina; Theileria species e.g. T.parvum and T.annulata; Cryptosporidium; and Toxoplasma gondii as well as the parasitic organism Pneumocystis carinii, and will therefore be useful in the treatment and/or prophylaxis of parasitic infections, such

as those caused by parasitic protozoa, eg. malaria, coccidiosis, cryptosporidiosis, toxoplasmosis and those caused by P.carinii eg. P.carinii pneumonia (PCP) in animals, including humans.

It will be appreciated that the amount of a compound of formula (I) or its salt required for use in the treatment or prophylaxis of the above-mentioned diseases will depend inter alia on the particular compound administered, the route of administration, the age and weight of the mammal (e.g. human) to be treated and the nature and severity of the condition being treated. In general, a suitable dose for administration to man for the treatment of malaria is in the range of 0.1mg to 200mg per kilogram bodyweight per day, for example from 1mg/kg to 100mg/kg, particularly 10 to 40 mg/kg. It will be appreciated that for administration to neonates, lower doses may be required.

For prophylactic treatment a compound of formula (I) or a salt thereof may also be given less frequently, e.g. as a single dose on alternate days, once or twice per week or once or twice per month. The dosage for prophylatic treatment will depend inter alia on the frequency of administration, and, where a depot preparation or controlled release formulation is used the rate of release of the active ingredient. Thus for once-weekly administration a suitable prophylactic dose is in the range 0.1 to 100 mg/kg,e.g. 0.5 to 50 mg/kg particularly 5 to 50 mg/kg.

It should be understood that for consistency the dosages referred to above are calculated in terms of the compound of formula (I) per se, and may require adjustment in the event a salt is employed.

The present invention thus further provides a method for the treatment and/or prophylaxis of parasitic infections e.g. parasitic protozoal infections such as malaria, or infections caused by P.carinii, in mammals e.g. humans, which comprises administering to a mammal suffering from or susceptible to said infection, an effective amount of a compound of formula (I) or a physiologically acceptable salt thereof.

There is also provided a compound of formula (I) or a physiologically acceptable salt thereof for use in therapy, e.g. in the treatment and/or prophylaxis of parasitic diseases as hereinbefore defined

The invention also provides the use of a compound of formula (I) or a physiologically acceptable salt thereof for the manufacture of a medicament for the treatment and/or prophylaxis of parasitic infections as hereinbefore defined.

For use according to the present invention a compound of formula (I) or a physiologically acceptable salt thereof is preferably presented as a pharmaceutical formulation. In general such pharmaceutical formulations will comprise a compound of formula (I) or a physiologically acceptable salt thereof together with one or more pharmaceutically acceptable carriers therefor and optionally other therapeutic and/or prophylactic ingredients. The carrier(s) must be acceptable in the sense of being compatible with the other ingredients of the formula and not deleterious to the recipient thereof.

The present invention, therefore, further provides a pharmaceutical formulation comprising a compound of formula (I) or a pharmaceutically acceptable salt thereof together with a pharmaceutically acceptable carrier therefor.

There is also provided a method for the preparation of a pharmaceutical formulation comprising bringing into association a compound of formula (I) or a pharmaceutically acceptable salt thereof, and a pharmaceutically acceptable carrier therefor.

A compound of formula (I) or its salt may conveniently be presented as a pharmaceutical formulation in unit dosage form. A convenient unit dose formulation contains a compound of formula (I) or a physiologically acceptable salt thereof in an amount of from 10 mg to 1g.

Pharmaceutical formulations include those suitable for oral, topical (including dermal, buccal and sublingual), rectal and parenteral (including subcutaneous, intradermal, intramuscular and intravenous) administration. The formulation may, where appropriate, be conveniently presented in discrete dosage units and may be prepared by any of the methods well known in the art of pharmacy. All methods include the step of bringing into association a compound of formula (I) or a physiologically acceptable salt thereof with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of the active ingredient(s). A tablet may be made by compression or moulding, optionally with one or more accessory ingredients. Compressed tablets may be prepared by compressing in a suitable machine the active ingredient(s) in a free-flowing form such as a powder or granules optionally mixed with a binder, lubricant, inert diluent, lubricating agent, surface-active agent or dispersing agent. Moulded tablets may be made by moulding in a suitable machine a mixture of the powdered active ingredient(s) with any suitable carrier. Tablets may be optionally coated and, if uncoated, may optionally be scored. Capsules may be prepared by filling the active ingredient(s) , either alone or in admixture with one or more accessory ingredients, into the capsule shells and then sealing them in the usual manner. Cachets are analogous to capsules wherein the active ingredient(s) together with any accessory ingredient(s) is (are) sealed in a rice paper envelope. A compound of formula (I) or a physiologically acceptable salt thereof may also be formulated as dispersible granules, which may for example be suspended in water before administration, or sprinkled on food. The granules may be packaged e.g. in a sachet. Formulations suitable for oral administration wherein the carrier is a liquid may be presented as a solution or a suspension in an aqueous liquid or a non-aqueous liquid, or as an oil-in-water liquid emulsion, e.g. a syrup, elixir, emulsion or a draught. A syrup may be made by adding the active ingredient(s) to a concentrated aqueous solution of a sugar, for example sucrose, to which may also be added any accessory ingredients which may include flavourings, agents to retard crystallisation of the sugar or agents which increase the solubility of other ingredients such as polyhydric alcohols for example glycerol or sorbitol.

Formulations for oral administration include controlled release dosage forms e.g. tablets wherein the active ingredient(s) is (are) formulated in an appropriate release - controlling matrix, or coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of the active ingredient(s) with the softened or melted carrier(s) followed by chilling and shaping in moulds.

Pharmaceutical formulations suitable for parenteral administration include sterile solutions or suspensions of the active ingredient(s) in aqueous or oleaginous vehicles. Injectible preparations may be adapted for bolus injection or continuous infusion. Such preparations are conveniently presented in unit dose or multi-dose containers which are sealed after introduction of the formulation until required for use. Alternatively, the active ingredient(s) may be in powder form eg. freeze-dried which is constituted with a suitable vehicle, such as sterile, pyrogen-free water, before use.

A compound of formula (I) or a physiologically acceptable salt thereof may also be formulated as a long-acting depot preparation, which may be administered by intramuscular injection or by implantation e.g. subcutaneously or intramuscularly.Depot preparations may include, for example, suitable polymerLc or hydrophobic materials, or Lon-exchange resins. Such long-acting fomulations are particularly convenient for prophylactic use.

Whilst the compounds of the invention may be formulated in any known manner, for example as described above, in view of their solubility they are particularly suited for formulation as aqueous solutions, especially for injection.

It should be understood that in addition to the aforementioned carrier ingredients the pharmaceutical formulations for the various routes of administration described above may include, as appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

The compounds of the present invention and the compounds of which they are pro-drugs i.e. compounds corresponding to formula (I) wherein $R^2$ is hydroxy may be administered in combination or concurrently with other therapeutic agents, for example other antimalarial agents, such as 4-aminoquinolines eg. chloroquine and amodiaquine; 8-aminoquinolines eg. primaquine; chloroquine; mefloquine; quinine; quinidine; artemesinin; artesumate; artemether; halofantrine; dihydrofolate reductase inhibitors eg. pyrimethamine; sulphonamides eg. sulphadoxine; proguanil; chloroproguanil; dapsone, hydroxynaphthoquinones; or mixtures eg. pyrimethamine/sulphadoxine; pyrimethamine/dapsone; and pyrimethamine/sulfalene; antibacterial agents such as trimethoprimsulphamethoxazole mixtures; anticoccidial agents such as monensin, halofuginone, arprinocid, amprolium, dinitolmide, robenidine or salinomycin; or antibiotics such as clindamycin, tetracycline, doxycycline, or spiromycin; or agents active against Pneumocystis carinii such as pentamidine or Eflornithine.

When compounds of formula (I) are used in combination with a second therapeutic agent the dose of each compound may vary from that required when the compound is used alone. Appropriate dosages can be readily determined by those skilled in the art.

Compounds of formula (I) may be prepared by reaction of a compound of formula (III)

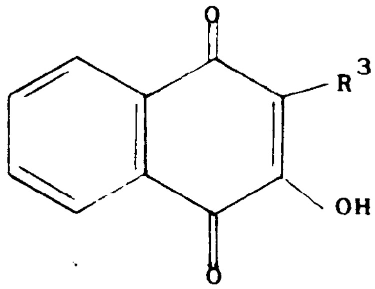

(III)

(wherein $R^3$ is as hereinbefore defined) with a phophorylating agent.

Phosphorylating agents which may be employed include phosphoric acid itself or more preferably derivatives of phosphoric acid in which at least one of the hydroxyl groups is replaced e.g. by a halogen atom or is esterified. Thus, suitable phosphorylating agents include phosphorus oxyhalides e.g. phosphorus oxychloride or oxybromide; phosphoric acid esters e.g. a mono-, di- or tri- ($C_{1-6}$ alkyl)phosphate, a mono- or di ($C_{1-6}$ alkyl) halophosphate p-nitrophenylphosphate or 2-(N,N-dimethylamino)-4- nitrophenylphosphate; phosphoric acid anhydrides e.g. pyrophosphoric acid or polyphosphoric acid; phosphorochloridates e.g. bis(4-nitro phenyl)phosphorochloridate or bis (β,β,β-trichloroethyl)phosphoro- chloridate; and cyclic phosphorylating agents e.g. 2-chloro-2-oxo-$P^v$-1,3,2-benzodioxophosphole or 2-methylthio-2-oxo-$P^v$-4H-1,3,2-benzodioxaphosphorine. Preferred phosphorylating agents are the phosphorus oxyhalides especially phosphorus oxychloride, and alkyl halophosphates e.g. diethylchlorophosphate.

The phosphorylation may conveniently be effected in the presence of a solvent which is inert to the reagents. Solvents which may be employed include aromatic hydrocarbons e.g. benzene or toluene; halogenated hydrocarbon e.g. chloroform or dichloromethane; ketones e.g. methylethylketone; amides e.g. dimethylformamide or hexamethylphosphoric triamide; ethers e.g. tetrahydrofuran or dioxan; pyridine; acetonitrile; trimethylphosphate and triethylphosphate. The reaction temperature may conveniently be in the range of from -80°C to +50°C, preferably -20°C to +10°C.

The reaction may advantageously be effected in the presence of a base which may be an organic base, for example pyridine, 4-dimethylaminopyridine, a tertiary amine such as triethylamine, or 1,8-diazabicyclo-[5,4,0]-7-undecene (DBU), or an inorganic base, for example an alkali metal carbonate such as potassium carbonate or sodium hydrogen carbonate.

To prepare a compound of formula (I) wherein $R^1$, $R^2$ and $R^4$ each represent a phosphate group the phosphorylation should be effected after reduction of the quinone nucleus, which may be effected in conventional manner.

It will be appreciated that where the phosphorylating agent is a phosphoric acid derivative in which at least two of the hydroxyl groups have been replaced as in e.g. phosphorus oxychloride, or substituted e.g. as in an alkyl phosphate it will be necessary, in order to obtain a compound wherein $R^1$ and $R^2$ are both hydrogen, to remove the halogen atoms or cleave the alkyl groups, which may be effected by a hydrolysis step, following the phosphorylation. The hydrolysis may be carried out by methods well known in the art, for example a mild aqueous hydrolysis will generally suffice to remove halogen atoms, and alkyl groups may be hydrolysed in an aqueous medium in the presence of a base. Alternatlvely the alkyl groups may be replaced in situ by a labile leaving group such as a trialkylsilyl e.g. trlmethylsilyl group, by reaction with an appropriate silylating agent, e.g. trlmethylsilyl chloride or bromide. The silyl groups may then be removed by alcoholysis, e.g. with methanol.

Salt formation can also be effected by methods well known in the art, by reacting a compound of formula (I) with an appropriate base.

Compounds of formula (III) may be prepared according to known methods for the synthesis of hydroxynaphthoquinone derivatives, such as are described in US Patents Nos. 2,553,648; 3,367,830; and 3,347,742; UK Patent No. 1,553,424; European Patents Nos. 2,228; 77,551; 77,550; and 123,238 and European Patent Application No. 362,996. Thus for example a compound of formula (III) may be prepared by reaction of a 2-halo (e.g. 2-chloro)-1,4-naphthoquinone with a compound seving to include the required $R^3$ group, followed by alkaline hydrolysis. Where $R^3$ is an optionally substituted cyclohexyl or optionally substituted cyclohexylmethyl group it may be introduced by reaction with the appropriately substituted cyclohexylcarboxylic acid or cyclohexylacetic acid.

The invention will now be further illustrated by the following nonlimiting examples:-

Example 1

3-[trans-4-(4-Chlorophenyl)cyclohexyl]-1,4-dioxo-2-naphthyldiethylphosphate

2-[trans-4-(4-Chlorophenyl)cyclohexyl]-3-hydroxy-1,4-naphthoquinone (4.2g), diethylchlorophosphate (4.2g) and sodium carbonate (1.6g) were mixed in 40ml of methylethylketone and refluxed for 1.5 hours. The yellow mixture was diluted with methylethylketone, washed with 80ml of 20% conc. hydrochloric acid and then with water. The methylethylketone solution (yellow) was dried ($MgSO_4$) and the solvent removed to give a yellow solid which was washed with ca.250ml 60-80°C petroleum ether (boiling) to give the title compound (3.52g).

m.p. 163-164°C.

$^1$H nmr ($CDCl_3$) δ 1.42 (6H, t of d, 2 x $CH_3$), 1.5-2.3 (8H, m, 4 x $CH_2$), 2.68 (1H, t of t, C$\underline{H}$($CH_2$)$_2$), 3.18 (1H, t of t C$\underline{H}$($CH_2$)$_2$), 4.40 (4H, m, 2 x $\underline{CH_2}CH_3$), 7.20 (4H, m, aromatic), 7.71 (2H, m, aromatic), 8.08 (2H, m, aromatic).

| | |
|---|---|
| Analysis: | Calculated for $C_{26}H_{28}ClO_6P$ |
| | C, 62.09; H, 5.61; Cl 7.05% |
| Found: | C, 61.65; H, 5.63; Cl 7.45% |

Example 2

3-[trans-4-(4-Chlorophenyl)cyclohexyl]-1,4-dioxo-2-naphthylphosphate

The product of Example 1 (2g) and trimethylsilybromide (5ml) were mixed in 20ml of carbon tetrachloride to give an orange solution which was left at room temperature overnight (ca.20°C, ca.20h). The solvent was removed to give an orange oil. Addition of methanol gave a yellow precipitate which was filtered off and washed with methanol. A further fraction was obtained by stripping the filtrate and washing the residue with further methanol. The solids so obtained were combined to give the title compound (1.43g).

m.p. 147-150°C.

$^1$H nmr ($Me_2SO-d_6$) 1.4-2.3 (8H, m, 4 x $CH_2$), 2.60 (1H, t of t, C$\underline{H}$($CH_2$)$_2$), 3.17 (1H, t of t, C$\underline{H}$($CH_2$)$_2$), 7.44 (4H, m, aromatic), 7.90 (2H, m, aromatic), 8.02 (2H, m, aromatic).

Example 3

3-[trans-4-(4-Chlorophenyl)cyclohexyl]-1,4-dioxo-2-naphthylphosphate bisdiethanolamine salt

A solution of diethanolamine (ca.60mg) in methanol (10ml) was added to a suspension of the product of Example 2 (104mg) in methanol (10ml) to give a bright yellow orange solution. The solvent was removed without heating to give an oil; ethyl acetate was added and the mixture scratched to give a yellow solid. The solid was filtered off and washed with ethyl acetate to give the title compound as a bright yellow solid (129mg).

decomp. ca 80°C

nmr - not recorded due to insolubility in $CDCl_3$ and $d_6$DMSO.

| | |
|---|---|
| Analysis: | Calculated for $C_{30}H_{42}ClN_2O_{10}P$ |
| | C 54.84; H 6.44; N 4.26; Cl 5.40% |
| Found: | C 54.60; H 6.53; N 4.07; Cl 5.08% |

Example 4

3-[trans-4-(4-Chlorophenyl)cyclohexyl]-1,4-dioxo-2-napthylphosphate, bismeglumine salt

The product of Example 2 (51mg) was dissolved in ethyl acetate (10ml) and N-methylglucosamine (meglumine) (36mg) added. The mixture was heated whereupon the meglumine dissolved and yellow, fluffy crystals of the salt prepipitated out. The crystals were filtered off and washed with ethyl acetate to give the title compound (55mg).

decomp ca 100°C
nmr - not recorded due to insolubility in $CDCl_3$ and DMSO.

| | | |
|---|---|---|
| Analysis: | Calculated for $C_{36}H_{54}ClN_2O_{16}P.H_2O$ |
| | C 50.56; H 6.60, N 3.28; Cl 4.15% |
| Found: | C 50.40; H 6.53; N 3.26; Cl 4.16% |

Example 5

In vivo Activity against Plasmodium yeolii in mice.

Test Compounds

Compound A: 3-[trans-4-(4-Chlorophenyl)cyclohexyl]-1,4-dioxo-2-naphthylphosphate bis diethanolamine salt.

Compound B: 3-[trans--4-(4-Chlorophenyl)cyclohexyl]-1,4-dioxo-2-naphthylphosphate N-methylglucosamine salt.

Compound (I): 2-[trans-4-(4-Chlorophenyl)cyclohexyl]-3-hydroxy-1,4-naphthoquinone.

Compounds A and B were administered orally and intravenously, and compound (I) was administered orally only, in the manner described below.

METHOD

The test method is a modified version of the 4-day suppressive test. The YM strain of P.yoelii was maintained in mice by twice weekly passage. Infected blood was collected and diluted with normal saline to give an inoculum of $3x10^6$ parasitized enythrocytes/ml. On the morning of Day 1 the test animals (male CD-1 mice weighing 18-20g) were infected intravenously via the tail vein with 0.1ml of inoculum/mouse.

Oral (p.o.)

Test compounds A, B and (I) were formulated by ball milling in 0.25% celacol with stainless steel balls. The total requirement was formulated at the beginning of a test and thereafter stored at 4°C.

Each test compound was administered orally (p.o.) to four or more usually five groups of mice, each group receiving a different dose level (dilution). A further group of mice was administered celacol only, as a control (ie. a total of 30 mice are used to obtain each $ED_{50}$). The mice were dosed orally in the late afternoon of Day 1 and in the early morning and late afternoon of days 2, 3 and 4, giving a total of seven doses in four days or 24 hours post-infection i.e. on day 2 when given as a single dose.

Intravenous (i.v.)

I.v. formulations of test Compounds A and B were prepared by dissolving the compound in water immediately before use, and administered via the tail vein as a single dose 24 hours post-infection, i.e. on day 2.

On the morning of day 5 smears of tail blood were prepared from each mouse and the parasitaemia counted. Data were analysed to yield $ED_{50}$ values by the best fit to a sigmoidal dose response curve. The results are presented in Table 1 below.

Results

Table 1

| | Dosing Schedule | | |
|---|---|---|---|
| | 7 x p.o. | 1 x p.o. | 1 x i.v. |
| Compound (I) | | | |
| $ED_{50}$ (mg/kg) | 0.03 | 0.09 | - |
| Compound (A) | | | |
| $ED_{50}$ (mg/kg) | 0.1<br>- | 0.5<br>0.17 | 0.57<br>0.17 |
| Weight adjusted $ED_{50}$ (mg/kg) | 0.056<br>- | 0.28<br>0.095 | 0.31<br>0.097 |
| Compound (B) | | | |
| $ED_{50}$ (mg/kg) | - | 0.23 | 0.27 |
| Weight adjusted $ED_{50}$ (mg/kg) | - | 0.10 | 0.12 |

The "weight adjusted" figure is the $ED_{50}$ of Compound A calculated in terms of the amount of compound of formula (I) administered, assuming the activity to be due to this component.

Example 6

The following examples illustrate with particular reference to the compound of Example 4 pharmaceutical formulations which may be employed in accordance with the present invention. It will be appreciated that other compounds of formula (II) may be formulated in similar manner.

A. Injectable solution

A solution for intramuscular injection may be prepared by mixing:-

| | | |
|---|---|---|
| Compound of Example 4 | 9.5 | parts by weight |
| Dimethyl sulphoxide | 19.0 | parts by weight |
| Sorbitan monooleate | 4.5 | parts by weight |
| Corn oil | 67.0 | parts by weight |
| | 100.0 | |

B. Injectable solution

| | | |
|---|---|---|
| Compound of Example 4 | 5 | parts by weight |
| N-methyl-pyrollidone | 48.3 | parts by weight |
| Tween 80 | 2 | parts by weight |
| Span 80 | 4.7 | parts by weight |
| Miglyol 812 | 40 | parts by weight |
| | 100.0 | |

C. Tablet

| | |
|---|---|
| Compound of Example 4 | 25.0 mg |
| Lactose BP | 48.5 mg |
| Microcrystalline Cellulose BP ("Avicel pH 101") | 10.0 mg |
| Low-substituted Hydroxypropyl; Cellulose BP ("LHPC LH-11") | 10.0 mg |
| Sodium Starch Glycollate BP ("Explotab") | 3.0 mg |
| Povidone BP ("K30") | 3.0 mg |
| Magnesium Stearate BP | 0.5 mg |
| | 100.0 mg |

D. IV Solution Freeze Dried

| | | |
|---|---|---|
| Compound of Example 4 | | 50mg |
| Water for Injections | to | 10ml |

Dissolve the naphthoquinone in the Water for Injections Sterilise by filtration. fill into glass vials and freeze dry Reconstitute shortly before use with Water for Injections.

E. Capsule

| | |
|---|---|
| Compound of Example 4 | 100 mg |
| Starch 1500 | 150 mg |
| Magnesium stearate | 2.5 mg |
| filled into a hard gelatin capsule | |

F. Aerosol Formulations

| | | | |
|---|---|---|---|
| a) | Compound of Example 4, micronised | | 1.0 mg |
| | Aerosol propellant | to | 5.0 ml |

Suspend the micronised compound in the aerosol propellant. Fill this suspension into preformed aerosol cannisters, 5 ml/cannister under pressure, through the valve orifice.

| | | | |
|---|---|---|---|
| b) | Compound of Example 4, micronised | | 1.0 mg |
| | Arlacel 85 | | 0.1% w/v |
| | Aerosol propellant | to | 5 ml |

Disperse the Arlacel 85 in the aerosol propellant and then add compound of Example 4. Fill the suspension into preformed aerosol cannisters, 5ml/cannister under pressure, through the valve orifice.

G. Powder Inhalation

| | |
|---|---|
| Compound of Example 4, micronised | 1.0 mg |
| Lactose | 29.0 mg |

Triturate and blend the micronised compound with the lactose. Fill the resulting powder blend into hard gelatin capsule shells, 30 mg per capsule.

## Claims

**1.** A compound of general formula (I)

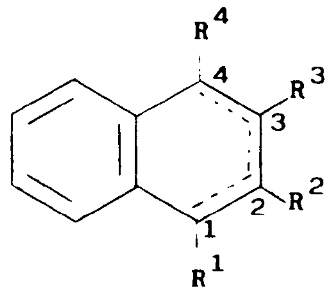

(I)

wherein

$R^3$ is $C_{1-35}$ non-aromatic hydrocarbon residue optionally substituted by one or more substituents, selected from halo, $C_{1-6}$alkoxy, hydroxy, phenyl, phenyl-$C_{1-6}$alkoxy and phenyl-$C_{1-6}$alkyl, each such phenyl group or moiety being optionally substituted by one or more groups selected from $C_{1-6}$alkoxy, $C_{1-6}$alkyl, $C_{1-6}$alkoxy-$C_{1-6}$alkyl, hydroxy, halogen, halo-$C_{1-6}$alkyl, amino, and mono-or di-$C_{1-4}$alkyl-amino; and either

the dotted line represents a double bond between the 2 and 3 positions of the naphthyl ring,

$R^1$ and $R^4$ each represent = 0, and

$R^2$ represents a phosphate group

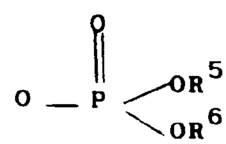

wherein $R^5$ and $R^6$, which may be the same or different, each represent a hydrogen atom or a $C_{1-6}$alkyl group;

or the dotted line represents double bonds between the 1, 2 and 3, 4 positions of the naphthyl ring, and

$R^1$, $R^2$ and $R^4$ each represent a phosphate group

$-OP(O)(OR^5)(OR^6)$

(wherein $R^5$ and $R^6$ are as hereinbefore defined);

and physiologically acceptable salts thereof.

2. A compound according to claim 1 wherein $R^3$ is a straight or branched chain $C_{1-14}$alkyl or $C_{2-14}$alkenyl group or a $C_{3-10}$cycloalkyl group, each of which may optionally carry a $C_{3-10}$cycloalkyl group, and each of the aforesaid cycloalkyl groups may optionally carry a $C_{1-10}$alkyl group.

3. A compound according to claim 1 or 2 wherein $R^3$ is

a $C_{1-10}$alkyl group;

a $C_{5-7}$cycloalkyl group which may be optionally substituted by a straight or branched chain $C_{1-6}$alkyl group, a halo-$C_{1-6}$alkyl group, a $C_{1-6}$alkoxy group or a phenyl group, the phenyl group itself being optionally substituted by one or more substituents selected from $C_{1-6}$alkyl and halogen; and

a $C_{1-10}$alkyl-$C_{5-7}$cycloalkyl group, wherein the cycloalkyl moiety may be optionally substituted as defined for the aforementioned $C_{5-7}$cycloalkyl group.

4. A compound according to any of claims 1 to 3 wherein $R^3$ is represented by the formula:

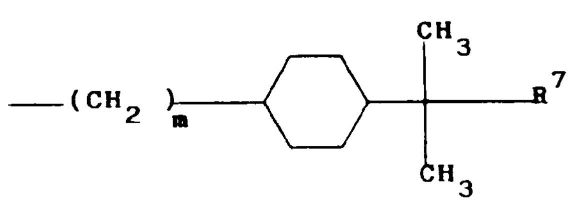

wherein:

m is zero or one and

$R^7$ is a $C_{1-10}$ alkyl group.

**5.** A compound according to any of claims 1 to 3 wherein $R^3$ is represented by the formula:

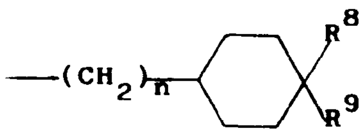

wherein:

n is zero or one, and

either

$R^8$ is hydrogen and

$R^9$ is selected from halogen, perhalo-$C_{1-6}$ alkyl, $C_{1-6}$ alkoxy, aralkoxy, $C_{1-6}$ alkyl-$C_{1-6}$ alkoxy, and phenyl substituted by one or two groups selected from halogen and $C_{1-6}$ alkyl

or

$R^8$ and $R^9$ are both $C_{1-6}$ alkyl or phenyl.

**6.** A compound according to any of claims 1 to 3 wherein $R^3$ is the 4-(4-chlorophenyl)cyclohexyl group:

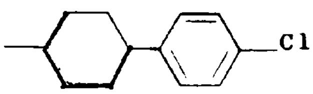

**7.** A compound according to any of claims 1 to 6 wherein $R^1$ and $R^4$ represent =O and the dotted line is a bond between the 2 and 3 positions of the naphthyl ring.

**8.** A compound according to claim 1 represented by the formula (II)

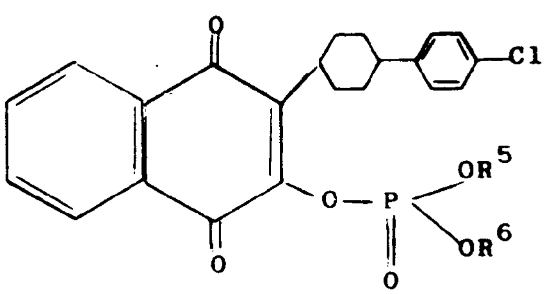

(II)

wherein $R^5$ and $R^6$ are as defined in claim 1

and physiologically acceptable salts thereof.

**9.** A compound according to claim 8 in the form of the trans isomer.

**10.** The meglumine salt of the compound according to claim 8 or claim 9 wherein both $R^5$ and $R^6$ represent hydrogen.

**11.** A method for the treatment and/or prophylaxis of parasitic infections in mammals which comprises administering to a mammal suffering from or susceptible to said infection, an effective amount of a compound of formula (I) or a physiologically acceptable salt thereof.

**12.** A compound of formula (I) or a physiologically acceptable salt thereof for use in therapy.

**13.** A compound of formula (I) for use in the treatment and/or prophylaxis of parasitic infections.

**14.** The use of a compound of formula (I) or a physiologically acceptable salt thereof for the manufacture of a medicament for the treatment and/or prophylaxis of parasitic infections.

**15.** A pharmaceutical formulation comprising a compound of formula (I) or a pharmaceutically acceptable salt thereof together with a pharmaceutically acceptable carrier therefor.

**16.** A method for the preparation of a pharmaceutical formulation comprising bringing into association a compound of formula (I) or a pharmaceutically acceptable salt thereof, and a pharmaceutically acceptable carrier therefor.

**17.** A unit dose formulation comprising as active ingredient a compound of formula (I) or a physiologically acceptable salt thereof in an amount of from 10 mg to 1g.

**18.** A process for the preparation of a compound of formula (I) comprising reaction of a compound of formula (III)

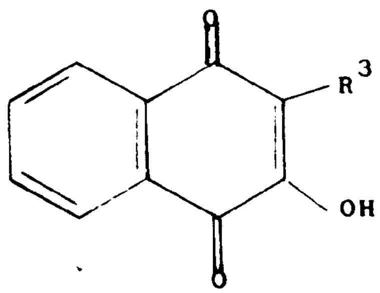

(III)

wherein $R^3$ is as defined in any of claims 1 to 6 with a phosphorylating agent.

**19.** A process according to claim 18 wherein the phosphorylating agent is a phosphorus oxyhalide, a phosphoric acid ester, a phosphoric acid anhydride, a phosphorochloridate, or a cyclic phosphorylating agent.

European Patent Office

# EUROPEAN SEARCH REPORT

Application Number
EP 92 30 9161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 362 996 (THE WELLCOME FOUNDATION LTD.)<br>* the whole document *<br>--- | 1,11-17 | C07F9/12<br>A61K31/66 |
| A | FR-A-886 334 (I.G.FARBENINDUSTRIE AKTIENGESELLSCHAFT)<br>* the whole document *<br>----- | 1,11-17 | |

TECHNICAL FIELDS SEARCHED (Int. Cl.5)
C07F
A61K

The present search report has been drawn up for all claims

| Place of search | Date of completion of the search | Examiner |
|---|---|---|
| THE HAGUE | 03 DECEMBER 1992 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)